# EUROPEAN PATENT APPLICATION

(11) **EP 2 990 480 A1**
(43) Date of publication of application: **02.03.2016**
(21) Application number: 14182939.0
(22) Date of filing: 29.08.2014
(51) Int. Cl.: C12N 9/06, C07K 16/40, A61K 39/395, A61P 35/00, A61K 39/00

(54) **Anti-LOXL4 antibodies and derivatives there**

(71) Applicant: Christian-Albrechts-Universität zu Kiel, 24118 Kiel (DE)
(72) Inventor: Görögh, Tibor, 24223 Schwentinental (DE)
(74) Representative: Steinecke, Peter

(57) **Abstract**

Provided are methods and compositions for the detection, prevention and treatment of primary and metastatic neoplastic diseases, including, but not limited to, human carcinomas, in particular head and neck squamous cell carcinoma. In particular, an antibody and antigen-binding fragment as well as variants thereof are provided that bind to an epitope of LOXL4 in the cytoplasm and/or on the cell surface of tumor cells, which are useful in the diagnosis and especially the treatment of a tumor which expresses LOXL4.

## Description

### FIELD OF THE INVENTION

The present invention relates to methods and compositions for the detection, prevention and treatment of primary and metastatic neoplastic diseases, including, but not limited to, human carcinomas, in particular head and neck squamous cell carcinoma (HNSCC). In accordance with the present invention, the practice of the detection, prevention and treatment of and cancer is mediated and/or indicated by the presence and localization of certain tumor markers in diseased tissue or cells. In particular, the present invention relates to antibodies and antigen binding molecules which are capable of binding to an epitope of lysyl oxidase-like 4 (LOXL4) that is localized in the cytoplasm and/or cell surface of tumor cells. Furthermore, the present invention relates to compositions comprising said antibodies and their use in methods of diagnosis and treating tumors. The present invention further concerns the use of an antibody capable of recognizing LOXL4 protein or a domain thereof for the detection and/or treatment of a tumor. Several documents are cited throughout the text of this specification. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

### BACKGROUND OF THE INVENTION

Lysyl oxidases are a family of five copper-dependent amine oxidases including LOX, LOXL, LOXL2, LOXL3 and LOXL4 that catalyze the oxidation of peptidyl lysine to δ-aminoadipic β-semialdehyde, the intermediate precursor during the formation of covalent cross-linkages that stabilize fibers of elastin and collagen, and contributes to the development and maintenance of the extracellular matrix (Kagan and Trackman 1991; Kagan and Li 2003; Mäki et al., 2001).

LOX is expressed in several human tumor cells like melanoma cells, fibrosarcoma and rhabdosarcoma cells, and in prostate- and breast carcinoma (Csiszar et al., 1996, 2001, 2002). It has been found in both extracellular and intracellular locations (Li et al., 1997; Mello et al., 1995), and has multiple functions in stromal and epithelial cells and tissues e.g., maturation of fibrillar matrix proteins in fibrosing processes and dictates their stability against metalloproteinases (Ren et al., 1998; Peyrol et al., 1997; Peyrol et al 2000). Studies investigating the role of LOX in invasive breast carcinoma cells revealed that LOX activity is essential in promoting the invasive phenotype of breast carcinoma cells (Kirschmann et al., 2002; Payne et al., 2005) and for metastatic behavior in mice (Erler et al., 2006). It was also demonstrated that hydrogen peroxide generated during LOX-catalyzed reactions mediates FAK/Scr activation, turnover of focal adhesions, and increases cell migration (Payne et al., 2005). A recent study showed that LOXL4 is induced by transforming growth factor β1 (TGF-ß1) and plays a role in ECM remodeling (Busnadiego et al., 2013).

Selective upregulation and amplification of the LOXL4 gene, as well as overexpression of LOXL4 protein was found in primary and metastatic head and neck squamous cell carcinoma (HNSCC) cell lines in comparison to normal epithelial cells as well as in patients with invasive HNSCC, correlating with local lymph node metastases versus primary tumor types and higher tumor stages (Görögh et al., 2007, Weise et al., 2008a). Furthermore, an affinity purified polyclonal antibody raised against the 93kD LOXL4 antigen was used to demonstrate LOXL4 expression in HNSCC tissues of different grading and staging, and to study its involvement in the structural organization of squamous epithelia (Weise et al., 2008b). Studies in cultured primary hypopharyngeal HTB-43 carcinoma cells detected perinuclear and cell surface expression of LOXL4, but no nuclear localization (Weise et al., 2008a). LOXL4 expression was further evaluated in a large cohort of patients (n = 257) with HNSCC whereby LOXL4 positive immunostaining was detected in almost all tissue samples obtained from primary tumors and lymph node metastases (Weise et al., 2008a). Among the samples of pathologically confirmed pre-malignant leukoplakia of the tongue and oral mucosa (n = 9), only those with a moderate to high degree of dysplasia stained positive for LOXL4. In all non-neoplastic tissue components (n = 9) and in those derived from 30 healthy controls containing mesenchymal tissues, keratinising, non-keratinising squamous and glandular epithelia, no LOXL4 immunoreactivity was detected (Weise et al., 2008a).

The analysis of LOXL4 gene organization and promoter region showed that TATA and the SP1 transcription factors are mainly involved in the upregulation of LOXL4 gene expression in HNSCC (Görögh et al., 2008). Furthermore, chromosome band 10q24.2, which contains the LOXL4 gene, is present in HNSCC cells in supernumerary copies of isochromosomes associating with LOXL4 overexpression (Görögh et al., 2007).

HNSCC is the sixth most common cancer worldwide, with almost 95% of the tumors originating from the mucosal squamous epithelium of the upper aerodigestive tract (Parkin et al., 1988). Unfortunately, local or regional disease recurs in one third of patients with advanced tumor stage, and distant metastases appear in 25% with a 5-year survival rate of around 40% despite aggressive bi-or trimodality standard treatments (Hoffmann et al., 1998). The availability of prognostic parameters or tumor antigens would allow the selection of patients for adjuvant treatment regimens as target therapies. Yet over the past decades, only limited success has been achieved in identifying unique targets or prognostic markers for TNM stage and behavior of tumor growth such as invasion depth and lymphangiosis carcinomatosa along with patients' characteristics (e.g., age, co-morbidity) in HNSCC.

So far, most of the described antibodies against HNSCC (Kimmel and Carrey 1986; Fernsten et al., 1986; Koprowska et al., 1986; Myoken et al., 1987; Ranken et al., 1987; Samuel et al., 1989; Tatake et al., 1989) showed considerable cross reactivity with normal tissue or showed only reactivity with SCC of distinct sites of origin (Gerretsen et al., 1991). The first successful radioimmunotherapy results for HNSCC were shown with the E48 mAb in the early 1990s (Gerretsen et al., 1992). Over the past two decades, much effort has been directed into developing mAbs like cetuximab (erbitux) which can block epidermal growth factor receptor (EGFR). This receptor is overexpressed in tumor cells and cetuximab has been approved for combination therapy with chemotherapy for HNSCC treatment (Bou-Assaly and Mukherji, 2010). Cetuximab was shown to inhibit the growth of 5 out of 8 (63%) xenograft tumors (Krumbach et al., 2011). Assuming that EGFR as a cell surface antigen is strongly overexpressed in head and neck carcinoma, the response rate of cetuximab is rather low.

The presence and localization of LOXL4 in tumor cells, in particular in HNSCC cells provides a valuable marker and may also be useful as a target for therapeutic intervention. It is thus highly desirable to have antibodies or other binding molecules, which specifically recognize epitopes of LOXL4 in the cytoplasm and/or the cell surface of such cells.

Thus, there is a need for a reliable source of anti-LOXL4 antibodies capable of detecting epitopes of LOXL4 and thereby enabling the specific detection and probably treatment of tumor cells.The solution to said technical problem is achieved by providing the embodiments characterized in the claims and described further below.

Bibliographic details of the publications referred to by author in this specification are collected alphabetically at the end of the description.

### SUMMARY OF THE INVENTION

The present invention relates to the technical field of immunology and the treatment of diseases mediated and/or indicated by the presence and localization of certain tumor markers on the cell surface of diseased tissue or cells. The present invention relates to an antibody or antigen-binding fragment thereof that binds to an epitope of LOXL4 that is localized in the cytoplasm and/or cell surface of tumor cells for use in the therapeutic treatment of a tumor which expresses LOXL4.

Particularly, the present invention concerns the use of a monoclonal antibody LOXL4-P as produced by hybridoma LOXL4-P, deposited with the DSMZ-Deutsche Sammlung von Mikroorganismen und Zellkulturen GmbH, Mascheroder Weg 1b, D-38124 Braunschweig, Germany on 19 March 2014, and assigned Accession Number DSM ACC3233. Furthermore, while the invention is illustrated and described by way of reference to the mouse monoclonal anti-LOXL4-P antibody DSM ACC3233 it is to be understood that the antibody or antibody fragment of the present invention include synthetic and biotechnological derivatives thereof, which means any engineered antibody or antibody-like LOXL4-binding molecule, synthesized by chemical or recombinant techniques, which retains one or more of the functional properties of the subject antibody, in particular its cytotoxic activity towards LOXL4-expressing cells. Thus, while the present invention may be described for the sake of conciseness by way of reference to the DSM ACC3233 antibody, unless stated otherwise synthetic and biotechnological derivatives thereof as well as equivalent LOXL4-binding molecules are meant as well and included with the meaning of the term antibody.

In a preferred embodiment, the antibody is a human, humanized, xenogeneic, or a chimeric human-murine antibody. Therapeutic compositions including the antibody or active fragments thereof, or agonists and cognate molecules, or alternately, antagonists of the same, and methods of use of such compositions in the prevention, diagnosis or treatment of tumorigenic diseases using these compositions are also included, wherein an effective amount of the composition is administered to a patient in need of such treatment. However, for diagnostic uses and research in general murine antibodies are preferred as well. The antigen-binding fragment of the monoclonal antibody can be a single chain Fv fragment, an Fab' fragment, an Fab fragment, and an F(ab')₂ fragment, or any other antigen-binding fragment. In a specific embodiment, infra, the monoclonal antibody or fragment thereof is a murine IgG or IgM isotype antibody.

Naturally, the invention extends to the hybridoma that produces monoclonal antibody LOXL4-P, which hybridoma is deposited with the DSMZ as indicated hereinbefore.

The present application also discloses polynucleotides encoding at least a variable region of an immunoglobulin chain of the antibody of the invention. Preferably, said variable region comprises at least one complementarity determining region (CDR) of the V_{H} and/or V_{L} of the variable region of the antibody LOXL4-P.

Accordingly, the present application also discloses vectors comprising said polynucleotides and host cells transformed therewith as well as their use for the production of an antibody capable of binding specifically extracellular localized epitopes of LOXL4-P on intact cells, in particular tumor cells, or a functional fragment or immunoglobulin chain(s) thereof.

It is also an object of the invention to provide bi- or multifunctional molecules that comprise a binding domain of an antibody, an immunoglobulin chain or a binding fragment of the present invention, which binds LOXL4, and at least one further functional domain.

The antibody, immunoglobulin chain(s), binding fragments thereof and ligands other than LOXL4 binding to said antibody can be used in pharmaceutical and diagnostic compositions for modulating and detecting an immune response or for the detection and/or treatment of a tumor.

Additionally, methods are provided for determining a tumor comprising assaying cells in a sample from a patient with the antibody or the bi- or multifunctional molecule according to the invention, wherein the presence or increased amount of LOXL4 is indicative for the tumor, and for treating a tumor in a subject in need thereof, comprising administering to the subject a therapeutically effective amount of said antibody or bi- or multifunctional molecule.
The use of the foregoing compositions in the preparation of medicament is preferred. In preferred embodiments, the medicament is useful in the treatment of conditions related to a tumor.

Further embodiments of the present invention will be apparent from the description and Examples that follow.

### BRIEF DESCRIPTION OF THE DRAWINGS

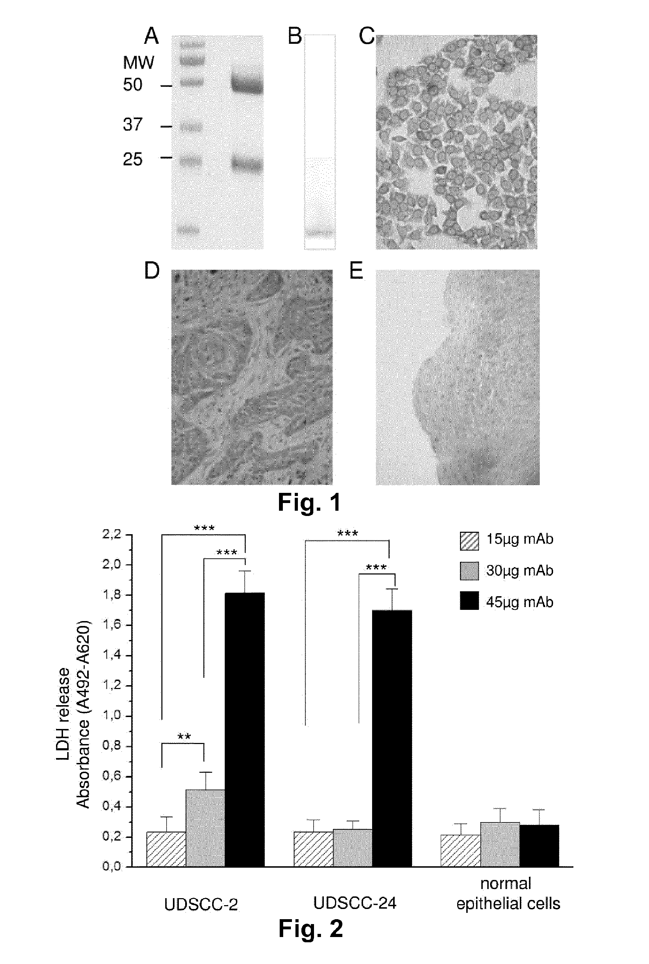

Figure **1****.** Analysis of the specificity of LOXL4-P. (A) SDS polyacrylamide gel electrophoresis representing the purity of the antibody (the 50 kD heavy chain and the 25 kD light chain are shown). (B) Western blot analysis for identifying LOXL4 peptide antigen with LOXL4-P. (C) LOXL4 reactivity in HNSCC cells and (D) in HNSCC biopsy. (E) No immunopositivity was seen in normal mucosa of the pharynx.
Figure **2****.** LDH release of cells after treatment with LOXL4-P. HNSCC cells and normal epithelial cells grown to a monolayer were incubated for 48 h in the presence of 15 µg/ml, 30 µg/ml, and 45 µg/ml of LOXL4-P mAb. LDH activity of cell free culture supernatant was determined as described (Example 3). Two asterisks represent P ≤ 0,001, and three asterisks represent P ≤ 0,0001.

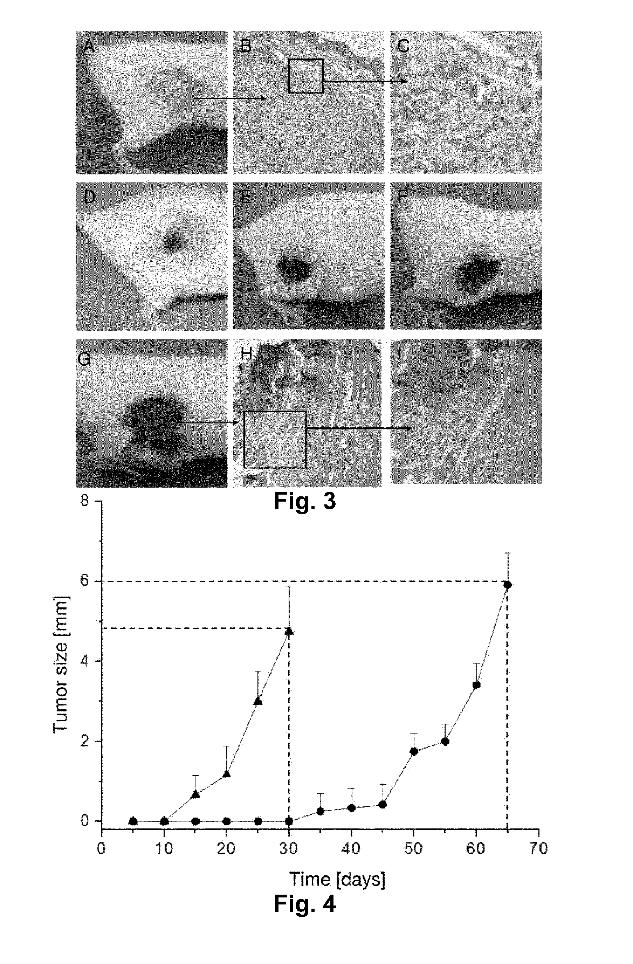

Figure **3****.** Macroscopical and histochemical appearances of xenograft tumors. (A) LOXL4-P-untreated tumor one month after tumor cell inoculation. (B and C) Densely packed tumor cell aggregates showing typical pattern of squamous cell carcinoma. (D - F) Tumor bearing mice were i.v. injected with LOXL4-P in weakly interval starting at tumor sizes of (D) 0,5 cm, (E) 0,8 cm, and (F) 1,2 cm respectively. Regardless of the tumor size, the necrotizing tumors become fully crusted. (G) Heavy crusted tumor residue was resected and prepared for histological observation (H and I). Microscopic cross-sections from G showing cell-free crusted wound layers.
**Figure. 4****.** Tumor development in SCID mice as a function of time. (A) After s.c. inoculation of mice (n = 12) with 10⁶ HNSCC cells the tumor grew quickly reaching a size of 0,5 cm over a period of 30 days. (●) Mice (n = 12) were pre-treated i.v. with 200 µg LOXL4-P. Immediately thereafter they were injected *s.c.* in the flank with 10⁶ HNSCC cells. Pre-treatment of animals with LOXL4-P considerably delayed tumor development.

### DETAILED DESCRIPTION OF THE INVENTION

The present invention concerns methods and compositions for the diagnosis, prevention and treatment of primary and metastatic neoplastic diseases in a human individual. In particular, the present invention relates to molecules that bind to an epitope of LOXL4 on tumor cells, for use in the treatment of a tumor which expresses LOXL4. More specifically, the present invention relates to the use of antibodies and antigen-binding fragments thereof, which demonstrate the immunological binding characteristics of monoclonal antibody LOXL4-P as produced by hybridoma LOXL4-P, deposited with the DSMZ-Deutsche Sammlung von Mikroorganismen und Zellkulturen GmbH, Mascheroder Weg 1b, D-38124 Braunschweig, Germany on 19 March 2014, and assigned Accession Number DSM ACC3233. Where present, the term "immunological binding characteristics," or other binding characteristics of an antibody with an antigen, in all of its grammatical forms, refers to the specificity, affinity, cross-reactivity, and other binding characteristics of an antibody.

The present invention provides a hybridoma line and the antibodies produced by it. It was surprisingly found that the purified antibody is capable of binding to LOXL4 in the cytoplasm and/or cell surface. Since only primary and metastatic HNSCC cells, but not the non-neoplastic tissue from adjacent sites of tumor samples, appear to express LOXL4, the antibodies according to the invention are able to distinguish between normal and tumor cells.
Thus, the invention provides an antibody or antigen-binding fragment thereof that binds to an epitope of LOXL4 of tumor cells, which gives rise to several embodiments described herein.
Since the expression of LOXL4 is a hallmark of several different types of cancer, a preferred embodiment of the antibody of the present invention binds to a tumor cell, wherein said tumor is a human tumor selected from the group consisting of head, neck, larynx, esophagus, stomach, lung, liver, colon, rectum, pancreas, breast, ovary, uterus, cervix, vulva, penis, bladder or kidney tumors.

In a preferred embodiment of the present invention, the antibodies recognize an epitope that comprises or consists of the amino acid sequence KVWDLKMR (SEQ ID NO: 1). Most preferably, said antibodies are monoclonal antibodies. In particular, the antibody or the antigen-binding fragment thereof of the present invention preferably exhibit the immunological binding characteristics of monoclonal antibody LOXL4-P as produced by hybridoma LOXL4-P, deposited with the DSMZ-Deutsche Sammlung von Mikroorganismen und Zellkulturen GmbH, Mascheroder Weg 1b, D-38124 Braunschweig, Germany on 19 March, and assigned Accession Number DSM ACC3233. The immunological binding characteristics of monoclonal antibody LOXL4-P are substantially the same as those of the polyclonal antibody described by Weise et al. (2008a). However, polyclonal antibody is a heterogeneous mixture of antibodies and it is not known beforehand whether a single kind, i.e. monoclonal antibody could or would exhibit the properties shown for the polyclonal antibody. Furthermore, while this publications by the inventors describes the desired immunological characteristics of an antibody of the present invention, especially that the antibodies are capable of binding to LOXL4-expressing HNSCC tumor cells, the present invention for the first time enables the unlimited provision of such antibodies and reliable sources, in particular the corresponding hybridoma cell line. Hence, the provision of the hybridoma producing monoclonal antibody LOXL4-P enables the person skilled in the art to design and produce functionally equivalent antibodies, for example by adapting the antigen-binding site of the mentioned antibody.

The antibody specifically provided is unique with respect to its immunological and biological activity. It may be distinguished from other anti-LOXL4 antibodies by its ability to bind to an epitope of LOXL4, in particular on intact and viable tumor cells. It is also capable of exhibiting an effect on cell death and cell lysis based on the measurement of LDH released from the cytosol of damaged cells into the supernatant (see Figure 2 and Table 2). Hence LOXL4-binding molecules derived from LOXL4-P are preferably used in but not limited to therapeutic and diagnostic applications.

Furthermore, in one embodiment the antibodies of the present invention are preferably characterized in that 1 to 100 µg/ml, preferably less than 50 µg/ml and most preferably about 15 to 45 µg/ml of the antibody of the invention is sufficient for cell death and cell lysis based on the measurement of LDH released from the cytosol of damaged cells into the supernatant. (see Figure 2 and Table 2). In such experiments usually 1 to 100 µg/ml, preferably 5 to 50 µg/ml, and most preferably about or less than 50 µg/ml of the given antibody is sufficient to obtain the same results as described in Figure 2 and Table 2.

Alternatively, the antibody of the present invention is a monoclonal antibody or antigen-binding fragment thereof, which competes for binding to an epitope of LOXL4 with an antibody provided by the present invention. Those antibodies may be murine as well; however, human, humanized, xenogeneic, or chimeric human-murine antibodies being preferred, in particular for therapeutic applications. An antigen-binding fragment of the antibody can be, for example, a single chain Fv fragment, an Fab' fragment, an Fab fragment, and an F(ab')₂ fragment. Thus, for some applications only the variable regions of the antibodies are required, which can be obtained by treating the monoclonal antibody isolated from the hybridoma with suitable reagents so as to generate Fab', Fab, or F(ab')₂ portions. Such fragments are sufficient for use, for example, in immunodiagnostic procedures involving coupling the immunospecific portions of immunoglobulins to detecting reagents such as radioisotopes.

Naturally, the invention extends to the hybridoma producing antibodies according to the invention as well. Thus, the invention advantageously provides an indefinitely prolonged cell source of a monoclonal antibody of the invention: the hybridoma. Particularly preferred is the hybridoma LOXL4-P, deposited with the DSMZ-Deutsche Sammlung von Mikroorganismen und Zellkulturen GmbH, Mascheroder Weg 1b, D-38124 Braunschweig, Germany on 19. March 2014, and assigned Accession Number DSM ACC3233.

As an alternative to obtaining immunoglobulins directly from the culture of hybridomas, the immortalized hybridoma cells can be used as a source of rearranged heavy chain and light chain loci for subsequent expression and/or genetic manipulation. Rearranged antibody genes can be reverse transcribed from appropriate mRNAs to produce cDNA according to methods known in the art, *e.g.* as described generally in the current editions of Molecular Cloning: A Laboratory Manual, (Sambrook et al., (1989) Molecular Cloning: A Laboratory Manual, 2nd ed., Cold Spring Harbor Laboratory Press). If desired, the heavy chain constant region can be exchanged for that of a different isotype or eliminated altogether. The variable regions can be linked to encode single chain Fv regions. Multiple Fv regions can be linked to confer binding ability to more than one target or chimeric heavy and light chain combinations can be employed. Once the genetic material is available, design of analogues as described above which retain both their ability to bind the desired target is straightforward. Methods for the cloning of antibody variable regions and generation of recombinant antibodies are known to the person skilled in the art.

In accordance with the above, the present application also discloses a polynucleotide encoding at least a variable region of an immunoglobulin chain of the antibody described above. Typically, said variable region encoded by the polynucleotide comprises at least one complementarity determining region (CDR) of the V_{H} and/or V_{L} of the variable region of the antibody produced by the above described hybridoma. The person skilled in the art knows that each variable domain (the heavy chain V_{H} and light chain V_{L}) of an antibody comprises three hypervariable regions, sometimes called complementarity determining regions or "CDRs" flanked by four relatively conserved framework regions or "FRs".

The antigen-binding domain formed by the positioned CDRs defines a surface complementary to the epitope on the immunoreactive antigen. This complementary surface promotes the noncovalent binding of the antibody to its cognate epitope. The amino acids comprising the CDRs and the framework regions, respectively, can be readily identified for any given heavy or light chain variable region by one of ordinary skill in the art, since they have been precisely defined; see, "Sequences of Proteins of Immunological Interest," Kabat, E., et al., U.S. Department of Health and Human Services, (1983); and Chothia and Lesk, J. Mol. Biol. 196 (1987), 901-917, which are incorporated herein by reference in their entireties. In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia and Lesk, J. Mol. Biol. 196 (1987), 901-917, which are incorporated herein by reference, where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular hypervariable region or CDR of the human IgG subtype of antibody given the variable region amino acid sequence of the antibody.

**Table 1: CDR Definitions¹**

| | **Kabat** | **Chothia** |
|---|---|---|
| VH CDR1 | 31-35 | 26-32 |
| VH CDR2 | 50-65 | 52-58 |
| VH CDR3 | 95-102 | 95-102 |
| VL CDR1 | 24-34 | 26-32 |
| VL CDR2 | 50-56 | 50-52 |
| VL CDR3 | 89-97 | 91-96 |

| | | |
|---|---|---|
| ¹Numbering of all CDR definitions in Table 1 is according to the numbering conventions set forth by Kabat *et al.* (see below). | | |

Kabat *et al.* also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody or antigen-binding fragment, variant, or derivative thereof of the present invention are according to the Kabat numbering system, which however is theoretical and may not equally apply to every antibody of the present invention. For example, depending on the position of the first CDR the following CDRs might be shifted in either direction.

Thus, the present invention also encompasses polypeptides and antibodies comprising at least one CDR of the above-described variable domain and which advantageously have substantially the same or similar binding properties as the antibody described herein above. The person skilled in the art will readily appreciate that using the variable domains or CDRs described herein antibodies can be constructed according to methods known in the art. Furthermore, the person skilled in the art knows that binding affinity may be enhanced by making amino acid substitutions within the CDRs or within the hypervariable loops which partially overlap with the CDRs as defined by Kabat (1983, 1987, 1990). Thus, the present invention also relates to antibodies wherein one or more of the mentioned CDRs comprise one or more, preferably not more than two amino acid substitutions.

The polynucleotide encoding the above described antibody may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Thus a vector comprising said polynucleotide, optionally in combination with a polynucleotide that encodes the variable region of the other immunoglobulin chain of said antibody is a preferred embodiment of the invention. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally polyA signals ensuring termination of transcription and stabilization of the transcript. Additional regulatory elements may include transcriptional as well as translational enhancers, and/or naturally associated or heterologous promoter regions. In this respect, the person skilled in the art will readily appreciate that the polynucleotide encoding at least the variable domain of the light and/or heavy chain may encode the variable domains of both immunoglobulin chains or only one. Likewise, said polynucleotides may be under the control of the same promoter or may be separately controlled for expression. Beside elements which are responsible for the initiation of transcription such regulatory elements may also comprise transcription termination signals. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or N-terminal identification peptide imparting desired characteristics, e.g., stabilization or simplified purification of expressed recombinant product. Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the immunoglobulin light chains, heavy chains, light/heavy chain dimers or intact antibodies, binding fragments or other immunoglobulin forms may follow.

Once the appropriate genetic material is obtained and, if desired, modified to encode an analogue, the coding sequences, including those that encode, at a minimum, the variable regions of the heavy and light chain, and inserted into an appropriate expression system, i.e. a vector which can be transfected, the antibody, or fragment thereof, may be expressed recombinantly in host cells. A host cell comprising a polynucleotide or a vector according to the invention is thus a preferred embodiment. Vectors may be plasmids, cosmids, viruses and bacteriophages used conventionally in genetic engineering. Apart from a polynucleotide encoding a variable domain of an immunoglobulin chain of the antibody of the invention, they may optionally comprise a polynucleotide of the invention that encodes the variable domain of the other immunoglobulin chain of the antibody of the invention. Preferably, said vector is an expression vector and/or a gene transfer or targeting vector. Expression vectors derived from viruses may be used for delivery of the polynucleotides or vector of the invention into targeted cell populations. Methods which are well known to those skilled in the art can be used to construct recombinant viral vectors. Alternatively, the polynucleotides and vectors of the invention can be reconstituted into liposomes for delivery to target cells. The vectors containing the polynucleotides of the invention (e.g., the heavy and/or light variable domain(s) of the immunoglobulin chains encoding sequences and expression control sequences) can be transferred into the host cell by well-known methods, which vary depending on the type of cellular host.

The present application furthermore discloses host cells transformed with a polynucleotide or vector of the invention. Said host cell may be a prokaryotic or eukaryotic cell. The polynucleotide or vector of the invention which is present in the host cell may either be integrated into the genome of the host cell or it may be maintained extrachromosomally. The host cell can be any prokaryotic or eukaryotic cell, such as a bacterial, insect, fungal, plant, animal or human cell. The term "prokaryotic" is meant to include all bacteria which can be transformed or transfected with DNA or RNA molecules for the expression of the antibody of the invention or the corresponding immunoglobulin chains. The term "eukaryotic" is meant to include yeast, higher plant, insect and preferably mammalian cells. Depending upon the host employed in a recombinant production procedure, the antibodies or immunoglobulin chains encoded by the polynucleotide of the present invention may be glycosylated or may be non-glycosylated. Antibodies of the invention or the corresponding immunoglobulin chains may also include an initial methionine amino acid residue. A polynucleotide of the invention can be used to transform or transfect the host using any of the techniques commonly known to those of ordinary skill in the art; see, *e.g.* the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). The genetic constructs and methods described therein can be utilized for expression of the antibody of the invention or the corresponding immunoglobulin chains in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Furthermore, transgenic animals, preferably mammals, comprising cells of the invention may be used for the large scale production of the antibody of the invention.

The present invention also provides a method for preparing an antibody that binds to an epitope of LOXL4 in the cytoplasm and/or cell surface of tumor cells, or a functional fragment or immunoglobulin chain(s) thereof, said method comprising (a) culturing a cell described above; and (b) isolating said antibody or functional fragment or immunoglobulin chain(s) thereof from the culture. The expression systems are preferably designed to include signal peptides so that the resulting antibodies are secreted into the medium; however, intracellular production is also possible. The transformed hosts can be grown in fermentors and cultured according to techniques known in the art to achieve optimal cell growth. Once expressed, the whole antibodies, their dimers, individual light and heavy chains, or other immunoglobulin forms of the present invention, can be purified according to standard procedures of the art. The antibody or its corresponding immunoglobulin chain(s) of the invention can then be isolated from the growth medium, cellular lysates, or cellular membrane fractions. The isolation and purification of the, e.g., microbially expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention. It will be apparent to those skilled in the art that the antibodies of the invention can be further coupled to other moieties for, e.g., drug targeting and imaging applications. Such coupling may be conducted chemically after expression of the antibody or antigen to the site of attachment or the coupling product may be engineered into the antibody or antigen of the invention at the DNA level. The DNAs are then expressed in a suitable host system, and the expressed proteins are collected and renatured, if necessary. Substantially pure immunoglobulins of at least about 90 to 95% homogeneity are preferred, and 98 to 99% or more homogeneity most preferred, for pharmaceutical uses. Once purified, partially or to homogeneity as desired, the antibodies may then be used therapeutically (including extracorporeally) or in developing and performing assay procedures.

The present invention also involves a method for producing cells capable of expressing an antibody of the invention or its corresponding immunoglobulin chain(s) comprising genetically engineering cells with the polynucleotide or with the vector of the invention. The cells obtainable by the method of the invention can be used, for example, to test the interaction of the antibody of the invention with its antigen.

As mentioned before, the immunoglobulin or its encoding cDNAs may be further modified. Thus, in a further embodiment the method of the present invention comprises any one of the step(s) of producing a chimeric antibody, humanized antibody, single-chain antibody, Fab-fragment, bi-specific antibody, fusion antibody, labelled antibody or an analog of any one of those. Corresponding methods are known to the person skilled in the art. A further source of antibodies to be utilized in accordance with the present invention are so-called xenogeneic antibodies. As discussed above, the antibody of the invention may exist in a variety of forms besides complete antibodies; including, for example, Fv, Fab and F(ab)₂, as well as in single chains.

The antibodies of the present invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Modifications of the antibody of the invention include chemical and/or enzymatic derivatizations at one or more constituent amino acids, including side chain modifications, backbone modifications, and N-and C-terminal modifications including acetylation, hydroxylation, methylation, amidation, and the attachment of carbohydrate or lipid moieties, cofactors, and the like. Likewise, the present invention encompasses the production of chimeric proteins which comprise the described antibody or some fragment thereof at the amino terminus fused to heterologous molecules such as an immunostimulatory ligand at the carboxyl terminus.

Additionally, the present invention encompasses small polypeptides including those containing a LOXL4 binding fragment as described above, for example containing the CDR3 region of the variable region of the mentioned monoclonal antibody. Such peptides may easily be synthesized or produced by recombinant means to produce a LOXL4 binding agent useful according to the invention. Such methods are well known to those of ordinary skill in the art. The sequence of the CDR regions, for use in synthesizing peptide LOXL4 binding agents, may be determined by methods known in the art. The heavy chain variable region is a peptide which generally ranges from 100 to 150 amino acids in length. The light chain variable region is a peptide which generally ranges from 80 to 130 amino acids in length. The CDR sequences within the heavy and light chain variable regions which include only approximately 3-25 amino acids may easily be sequenced by one of ordinary skill in the art. To determine whether a peptide binds to LOXL4 any known binding assay may be employed.

Screening of LOXL4 binding agents also can be carried out utilizing a competition assay. If the LOXL4 binding agent being tested competes with the anti-LOXL4 monoclonal antibody of the present invention, as shown by a decrease in binding of the monoclonal antibody, then it is likely that the agent and the anti-LOXL4 monoclonal antibody bind to the same, or a closely related, epitope. Still another way to determine whether an agent has the specificity of the anti-LOXL4 monoclonal antibody described above is to pre-incubate the monoclonal antibody with LOXL4 with which it is normally reactive (i.e. binds), and then add the agent being tested to determine if the agent being tested is inhibited in its ability to bind LOXL4. If the agent being tested is inhibited then, in all likelihood, it has the same or a functionally equivalent epitope and specificity as the anti-LOXL4 monoclonal antibodies.

The polypeptides (e.g., antibodies) and other LOXL4 binding agents described above can also be used immunotherapeutically for disorders in humans. The term "immunotherapeutically" or "immunotherapy" as used herein in conjunction with the LOXL4 binding agents denotes both prophylactic as well as therapeutic administration. Thus, the peptides can be administered to high-risk subjects in order to lessen the likelihood and/or severity of a disease such as a malignant tumor, or administered to subjects already evidencing such disease.

Hence, the present invention relates to any antibody and similar binding molecules, which preferably have substantially the same immunological binding characteristics as monoclonal antibody LOXL4-P, i.e. which recognize the same epitope and with substantially the same affinity, or at least 1/10 of the affinity as the antibody of the invention exemplified herein. Such antibodies and binding molecules can be tested for their binding specificity and affinity by for example by using competitive assays with an antibody produced by the hybridoma of the invention. The antibodies of the present invention will typically find use individually in treating substantially any disease susceptible to monoclonal antibody based therapy.

As described above, the polynucleotide of the invention can be used alone or as part of a vector to express the (poly)peptide of the invention in cells. In principle this also enables gene therapy of diseases related to inappropriate expression of LOXL4.

Membrane LOXL4 may interact with other cell surface molecules, and it is reasonable to assume that agents modulating these interactions will have beneficial, additive and preferably synergistic effects on the treatment of diseases and conditions, wherein either of these proteins are involved in. Furthermore, such agents are expected to be useful in diagnosis, where the presence or absence of either of said proteins is associated with said disease or condition. Accordingly, the present invention also provides novel bi- or multifunctional molecules that comprise the binding domain of an antibody according to the invention, an immunoglobulin chain thereof or a binding fragment thereof which bind LOXL4, and at least one further functional domain; see also supra. In a preferred embodiment said bi- or multifunctional molecule is a bispecific molecule, particularly preferred a bispecific antibody.
The term "bispecific molecule" includes molecules which have at least the two mentioned binding domains directly or indirectly linked by physical or chemical means. However, the bispecific molecule of the present invention may comprise further functional domains such as additional binding domains and/or moieties such as a cytotoxic agent or a label and the like. In a preferred embodiment, the bispecific molecule of the present invention is a bispecific antibody. The bispecific antibodies may comprise Fc constant regions, for example for association of the polypeptide chains comprising the binding domains. In addition to providing for association of the polypeptide chains, Fc constant domains contribute other immunoglobulin functions. The functions include activation of complement mediated cytotoxicity, activation of antibody dependent cell-mediated cytotoxicity and Fc receptor binding. When antigen-binding proteins of the invention are administered for treatment or diagnostic purposes, the Fc constant domains can also contribute to serum half-life. The Fc constant domains can be from any mammalian or avian species. When antigen binding proteins of the invention are used for treatment of humans, constant domains of human origin are preferred, although the variable domains can be non-human. In cases where human variable domains are preferred, chimeric scFvs can be used.

Furthermore, the present invention relates to a composition comprising the antibody, the bi- or multifunctional molecule, the polynucleotide or the above described vector or cell of the invention. The composition of the present invention may further comprise a pharmaceutically acceptable carrier. Additionally, moieties may be added that improve the solubility, half-life, absorption, etc. of the base molecule. Alternatively the moieties may attenuate undesirable side effects of the base molecule or decrease the toxicity of the base molecule. Examples of suitable pharmaceutical carriers are well known in the art and include phosphate buffered saline solutions, water, emulsions, such as oil/water emulsions, various types of wetting agents, sterile solutions, etc.. Compositions comprising such carriers can be formulated by well-known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Aerosol formulations such as nasal spray formulations include purified aqueous or other solutions of the active agent with preservative agents and isotonic agents. Such formulations are preferably adjusted to a pH and isotonic state compatible with the nasal mucous membranes.
The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0,001 to 1000 mg per kilogram of body weight; however, doses below or above this exemplary range are envisioned, especially considering the aforementioned factors. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils.

Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like. Furthermore, the pharmaceutical composition of the invention may comprise further agents depending on the intended use of the pharmaceutical composition. Furthermore, the pharmaceutical composition may also be formulated as a vaccine, for example, if the pharmaceutical composition of the invention comprises a bispecific molecule described above for passive immunization.
Therapeutic or diagnostic compositions of the invention are administered to an individual in a therapeutically effective dose sufficient to treat or diagnose disorders as mentioned above. The effective amount may vary according to a variety of factors such as the individual's condition, weight, sex and age. Other factors include the mode of administration. The pharmaceutical compositions may be provided to the individual by a variety of routes such as by intracoronary, intraperitoneal, subcutaneous, intravenous, transdermal, intrasynovial, intramuscular or oral routes. In addition, co-administration or sequential administration of other agents may be desirable. A therapeutically effective dose refers to that amount of active molecule of the invention sufficient to ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The pharmaceutical composition of the present invention further comprises an immune stimulatory agent in a preferred embodiment. Immune stimulatory agents are used to enhance an immune reaction or to induce an immune reaction against epitopes which do not trigger an humoral or cytotoxic defense reaction under normal conditions. Such agents are well known in the art and can be chosen from a wide variety of molecules such as co-stimulatory molecules, e.g., cytokines and/or adjuvants. An "adjuvant" refers to a substance that enhances an immune response, including, for example, but not limited to, an antigen's immune-stimulating properties or the pharmacological effect(s) of a compound or drug. An adjuvant may nonspecifically enhance an immune response, e.g., the immune response to an antigen. "Freund's Complete Adjuvant," for example, is an emulsion of oil and water containing an immunogen, an emulsifying agent and mycobacteria. An adjuvant may comprise oils, emulsifiers, killed bacteria, aluminum hydroxide, or calcium phosphate (e.g., in gel form), or combinations thereof. An adjuvant may be administered into a subject (e.g., via injection intramuscularly or subcutaneously) in an amount sufficient to produce antibodies.

The present application also discloses a diagnostic composition comprising an antibody, a bi-or multifunctional molecule, a polynucleotide, a vector or a cell according to the invention, and optionally reagents conventionally used in immuno- or nucleic acid based diagnostic methods. For use in diagnosis, a variety of techniques are available for labeling biomolecules, many different labels, methods of labeling and detection are well known to the person skilled in the art. In addition, the above described compounds etc. may be attached to a solid phase. Suitable methods of immobilizing bispecific molecules of the invention on solid phases include but are not limited to ionic, hydrophobic, covalent interactions and the like.

The present application also discloses to a kit comprising an antibody or a bispecific molecule of the invention. Such kits are useful for a variety of purposes including but not limited to forensic analyses, diagnostic applications, and epidemiological studies in accordance with the above described diseases and disorders. Such a kit would typically comprise a compartmentalized carrier suitable to hold in close confinement at least one container. The carrier would further comprise reagents for detection such as labeled antigen or enzyme substrates or the like.

Naturally the present invention also encompasses a method of diagnosing a tumor comprising assaying cells in a sample from a patient with the antibody, or the bi- or multifunctional molecule according to the invention, wherein the presence or increased amount of LOXL4 is indicative for the tumor. This method preferably comprises an immunological step. Commonly used diagnostic methods employing antibodies and/or bi- or multifunctional molecules are for example immunohistochemistry on frozen or paraffin embedded tissue sections, Western blots, immunoprecipitation, etc.. Optionally, easy to detect signal producing agents can be used in conjunction with said antibodies or bi- or multifunctional molecules. Detectable signal-producing agents are useful *in vivo* and *in vitro* for diagnostic purposes. The signal producing agent produces a measurable signal which is detectable by external means, usually the measurement of electromagnetic radiation. For the most part, the signal producing agent is an enzyme or chromophore, or emits light by fluorescence, phosphorescence or chemiluminescence.

As described before, the compositions of the present invention are useful in diagnosis, prophylaxis, vaccination or therapy. Accordingly, the present invention relates to the use of the antibody, the bi- or multifunctional molecule, the nucleic acid molecule or the cell of the present invention for the preparation of a pharmaceutical composition for the treatment of a tumor. For these embodiments, the antibodies or the bi- or multifunctional molecules of the invention can be chemically or biosynthetically linked to anti-tumor agents or detectable signal-producing agents. Antitumor agents linked to a bispecific molecule, for example a bispecific antibody, include any agents which destroy or damage a tumor to which the antibody has bound or in the environment of the cell to which the antibody has bound. For example, an anti-tumor agent is a toxic agent such as a chemotherapeutic agent or a radioisotope. A method of treating a tumor in a subject in need thereof usually comprises administering to the subject a therapeutically effective amount of the antibody or the bi- or multifunctional molecule. As mentioned above, a therapeutically effective dose refers to that amount of active molecule of the invention sufficient to ameliorate the symptoms or condition. Therapeutic efficacy and toxicity of such compounds can be determined by standard pharmaceutical procedures in cell cultures or experimental animals. For the purpose of this invention said pharmaceutical composition is preferably designed to be administered intravenously, intramuscularly, subcutaneously, intraperitoneally, or as an aerosol.

Preferably, the tumor to be treated or diagnosed is selected from the group consisting of carcinomas of head, neck, larynx, esophagus, stomach, lung, liver, colon, rectum, pancreas, breast, ovary, uterus, cervix, vulva, penis, bladder or kidney, and metastatic cells in general.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease.

Furthermore, the term "subject" as employed herein relates to animals in need of amelioration, treatment and/or prevention of a neoplastic or infectious disease. Most preferably said subject is a human.

Due to the expression of LOXL4 on the surface of disease related cells, the invention provides the means for the targeted delivery to these cells while avoiding the normal cells. This is of particular advantage if toxic moieties are linked to a therapeutic molecule. Such a method of targeting a therapeutic and/or diagnostic agent to a cell which expresses an epitope of LOXL4 on the cell surface comprises administering to the subject a therapeutically effective amount of a bi- or multifunctional molecule of the invention. The use of a bi- or multifunctional molecule for targeting a therapeutic and/or diagnostic agent to a cell which expresses an epitope of LOXL4 on the cell surface is, of course, a preferred embodiment of this invention. Said targeted cell can be preferably a tumor cell.

From the foregoing, it is evident that the present invention encompasses any use of a ligand binding molecule comprising at least one CDR of the above described antibody, in particular for diagnosing and/or treatment of a disorder related to the expression or malfunction of LOXL4 in the cytoplasm and/or on the cell surface of target cells, in particular tumor cells. Preferably, said ligand binding molecule is an antibody of the present invention or an immunoglobulin chain thereof.

In addition, the present invention relates to anti-idiotypic antibodies of the mentioned monoclonal antibody described hereinbefore. These are antibodies or other binding molecules which bind to the unique antigenic peptide sequence located on an antibody's variable region near the antigen binding site. One concept for immune therapy of cancer involves induction of antigen mimic antibodies to trigger the immune system into a response against the tumor cells. Anti-idiotypic antibodies (Ab2) directed against the antigen-combining site of other antibodies (Ab1) may functionally and even structurally mimic antigen and induce anti-anti-idiotypic immune response. Preferably, the anti-idiotypic antibody is humanized.

The biological activity of the antibodies identified here suggests that they have sufficient affinity to make them potential candidates for drug localization to cells expressing the appropriate surface structures. This targeting and binding to cells could be useful for the delivery of therapeutically or diagnostically active agents (including targeting drugs, DNA sequences, RNA sequences, lipids, proteins (e.g., human growth factors)) and gene therapy/gene delivery. Molecules/particles with an antibody of the invention would bind specifically to cells/tissues expressing LOXL4, and therefore could have diagnostic and therapeutic use. Thus, the antibody or the antigen of the present invention can be labeled (e.g., fluorescent, radioactive, enzyme, nuclear magnetic) and used to detect specific targets *in vivo or in vitro* including "immunochemistry"-like assays *in vitro. In vivo* they could be used in a manner similar to nuclear medicine imaging techniques to detect tissues, cells, or other material expressing LOXL4, in particular on the cell surface of target cells. Another method involves delivering a therapeutically active agent to a patient. The method includes administering at least one antibody or the antigen-binding fragment and the therapeutically active agent to a patient. Preferably, the therapeutically active agent is selected from drugs, DNA sequences, RNA sequences, proteins, lipids, and combinations thereof. More preferably, the therapeutically active agent is an antibacterial agent, anti-inflammatory agent, or antineoplastic agent. The therapeutically or diagnostically active agent can be coupled to the antibody of the invention or an antigen-binding fragment thereof by various means.
Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins.
The invention further contemplates linking molecules of the invention to target or reporter moieties. Target moieties are first members of binding pairs. Anti-tumor agents, for example, are conjugated to second members of such pairs and are thereby directed to the site where the antigen-binding protein is bound.
By a further embodiment as mentioned before, the ligand binding molecules and antibodies of the invention may also be used in a method for the diagnosis of LOXL4-related diseases in an individual by obtaining a body fluid sample from the tested individual which may be a blood sample, a lymph sample or any other body fluid sample and contacting the body fluid sample with an antibody of the invention under conditions enabling the formation of antibody-antigen complexes. Similarly, biopsy or other specimen may be taken common in tumor diagnostic. The level of such complexes is then determined by methods known in the art, a level significantly higher than that formed in a control sample indicating the disease in the tested individual. In the same manner, the specific antigen bound by the antibodies of the invention may also be used. Thus, the present invention relates to an *in vitro* immunoassay comprising the antibody or the antigen of the invention.

Unless otherwise stated, a term and an embodiment as used herein is given the definition as provided and a common term as used herein is given the definition as provided in the Oxford Dictionary of Biochemistry and Molecular Biology, Oxford University Press, 1997, revised 2000 and reprinted 2003, ISBN 0 19 850673 2. These and other embodiments are disclosed and encompassed by the description and examples of the present invention.

The above disclosure generally describes the present invention. A more complete understanding can be obtained by reference to the following specific examples.

### EXAMPLES

The following examples are intended to illustrate the present invention but not to limit the scope thereof.

### Example 1: Establishment of monoclonal antibodies (mAbs) specific for LOXL4

Three seven week old female Balb/c mice obtained from Charles River (Sulzfeld, Germany) were immunized with 20 µg Keyhole-Limpet-Hemocyanin (KLH)-conjugated LOXL4 specific peptide antigen located within the second of four N-terminal scavenger receptor cysteine-rich (SRCR) domains of LOXL4 (Mäki et al., 2001). Mice were boosted four times by s.c. injection with 5 µg antigen at 10-day intervals. Prior to cell fusion a consecutive 3-day challenge of 3 µg antigen was injected intraperitoneally. The LOXL4 peptide-specific immune response was monitored during the immunization procedure by ELISA. Spleen cells of mice producing LOXL4 specific mAbs were fused with Ag8 653 cells according to the protocol by Köhler and Milstein (1975). About three weeks after fusion, supernatants of growing clones were tested for LOXL4 antigen-specific antibody response by ELISA. Positive clones were tested on LOXL4 expressing HNSCC cells and HNSCC tissues. For the therapeutic studies LOXL4-P was purified from the supernatants of the hybridoma cell cultures by means of immobilized HiTrap Protein-G HP column using the BioLogic Duo Flow FPL Chromatography System (Bio-Rad, Germany). Purified mAb was lyophilized, dissolved in PBS and the protein concentration was determined by the Bradford method (Bradford, 1976).

### Example 2: HNSCC cell sensitivity to LOXL4-P

### Human cell lines

For the *in vitro* experiments cell lines derived from human HNSCC of different localizations were used: esophagus (UKHN-2), tongue (UKHN-3, 7, UTSCC-24, and UPCI-SCC-154), tonsil (UKHN-6, 8, 9, and UMSCC-81B), mouth (UTSCC-45), larynx (UMSCC-10B, 24, UTSCC-9, 19A, and 23) pharynx (HTB-43), and hypopharynx (UD-2). All cell lines were genotyped by single tandem repeat DNA typing. Five primary human epithelial cell- and fibroblast cultures, derived from normal mucosa of the pharynx and the larynx served as controls. Prior to analyses, all cell lines were negatively tested for mycoplasma. Normal mucosa biopsies were retrieved during surgery after written patient consent was obtained, in accordance with the Ethical Commission of the University Hospital of Schleswig-Holstein, Campus Kiel, subjected to the Helsinki Declaration, revised 1983 (No. AZ D438/10). Carcinoma cells were grown in minimum essential medium with 10 % (v/v) fetal calf serum (Biochrom, Berlin, Germany) at 37 °C in 5 % CO₂ humidified atmosphere. Normal epithelial cells were grown in SFM-medium (LifeTechnologies, Inc. Eggenstein, Germany) under the same conditions.

### Cytotoxicity assay

Quantification of cell death and cell lysis was based on the measurement of LDH released from the cytosol of damaged cells into the supernatant using a non-radioactive LDH detection kit (Roche Diagnostics, Germany). HNSCC cells, normal epithelial cells, and normal fibroblasts grown to monolayers were incubated for 48 h in the presence of 15 µg/ml, 30 µg/ml, and 45 µg/ml or absence of LOXL4-P. After centrifugation at 250 x g for 10 min the cell-free culture supernatants were collected from LOXL4-P treated and control cells and incubated according to the manufacturer's instruction. To calculate percent cytotoxicity, appropriate controls were measured in each experiment according to the manufacturer's instructions. Absorbance was measured at 492 nm and 620 nm using a 96-well plate ELISA reader (Dynatech MR5000, Denkendorf, Germany).

### Clonogenic assay

At day 0, HNSCC cells, normal epithelial cells, and normal fibroblasts were plated in duplicate in 6-well plates. One week later, after cells had reached confluency, they were incubated for 48 h, 72 h and 96 h at various LOXL4 P-concentrations (15 µg/ml, 30 µg/ml, 45 µg/ml). Subsequently, cells were washed with PBS, fixed in ethanol and stained with crystal violet (0,1 % crystal violet in 20 % ethanol). Stained cells were measured by microscopic counting of 10 randomly chosen middle power magnification (x 200) fields. LD50 values were calculated and presented as mean plus/minus standard deviations (SD).

FPLC purified monoclonal antibody (mAb) LOXL4-P was tested for LOXL4 antigen specific antibody response, and on LOXL4 expressing HNSCC cells and HNSCC tissues (Figure 1). To elucidate the cytotoxic effect of LOXL4-P, several HNSCC cell lines originated from head and neck tumors of different anatomical locations were analyzed in comparison to normal epithelial cells and fibroblasts (Table 2). Of the 17 tumor target cell lines 13 (76%) were highly sensitive to LOXL4-P. The median lethal dose, LD50, was reached at concentrations of 15 µg/ml (UKHN-3, -6, -9, UMSCC-81B, UTSCC-19A), 30 µg/ml (HTB-43, UKHN-2, -7, UTSCC-9,-24), and 45 µg/ml (UDSCC-2, UMSCC-24, UPCI-SCC-154), respectively, over a period of 48 hours. The remaining 4 carcinoma cell lines (UKHN-8, UMSCC-10B, UTSCC-23, UTSCC-45) proved not to be sensitive to LOXL4-P or showed negligible (< 20%) cytotoxicity under the same experimental conditions, indicating differences between HNSCC cell lines regarding sensitivity to LOXL4-P. Based on these LD50 values, normal epithelial cells and fibroblasts were more resistant to LOXL-P than most HNSCC cells. A concentration of 45 µg/ml LOXL4-P was sufficient for maximum cytotoxicity in HNSCC cell lines whereas normal epithelial cells and fibroblasts showed no responses to this LOXL4-P concentration. However, concentrations higher than 50 µg/ml had an inhibitory effect on the proliferation of normal cell lines (data not shown). Intriguingly, the damaging effect of LOXL4-P on the tumor cells correlated with the release of the LDH enzyme, as exemplarily shown in Figure 2. Morphologic characteristics of the carcinoma cells in the presence of different LOXL4-P concentrations changed in a dose-dependent manner. The typical evidences of cell damage were structural changes in cell size, shape, and appearance. Complete destruction of carcinoma cells was indicated by cellular disintegration and detachment from the culture surface.

**Tab. 2 Effect of LOXL4-P on HNSCC cells and normal cells**

| No. | Target cells | Tumor localization | Type of Lesion* | Staging | Grading | Cytotoxic effect of LOXL4-mAb** | | |
|---|---|---|---|---|---|---|---|---|
| | | | | | | 15µg | 30µg | 45µg |
| 1 | UKHN-3 | Tongue | P | T4N1M0 | G2 | yes | | |
| 2 | UKHN-6 | Floor of the mouth | P | T3N1M0 | G2 | yes | | |
| 3 | UMSCC-10B | Larynx | M | T3N1M0 | G3 | yes | | |
| 4 | UMSCC-81B | Tonsil | P | T2N0M0 | G2 | yes | | |
| 5 | UTSCC-19A | Larynx | P | T4N0M0 | G2 | yes | | |
| 6 | HTB-43 | Pharynx | P | unknown | G1 | | yes | |
| 7 | UKHN-2 | Esophagus | P | T4N3M1 | G2 | | yes | |
| 8 | UKHN-7 | Tongue | P | T2N2M0 | unknown | | yes | |
| 9 | UTSCC-9 | Larynx | P | T4N0M0 | G1 | | yes | |
| 10 | UTSCC-24 | Tongue | P | T2N0M0 | G2 | | yes | |
| 11 | UDSCC-2 | Hypopharynx | P | T1N3M0 | G3 | | | yes |
| 12 | UMSCC-24 | Larynx | P | T2N0M0 | unknown | | | yes |
| 13 | UPCI-SCC-154 | Tongue | P | T4N2M0 | G3 | | | yes |
| 14 | UKHN-8 | Tonsil | P | T1N2M0 | unknown | | | no |
| 15 | UKHN-9 | Tonsil | P | T4N0M0 | unknown | | | no |
| 16 | UTSCC-23 | Larynx | P | T3N0M0 | | | | |
| 17 | UTSCC-45 | Floor of the mouth | P | T3N1M0 | G3 | | | no |
| 1-5 | Normal epitheial cells | Mucosa*** | - | - | - | | | no |
| 1-5 | Normal fibroblasts | Mucosa | - | - | - | | | no |

| | | | | | | | | |
|---|---|---|---|---|---|---|---|---|
| * P (Primary), M (Metastasis). ** Cytotoxic effect was identified as the LD50 value (i.v.). *** Normal epithelial cells and fibroblasts served as controls. They were derived from normal mucosa of the larynx and pharynx (see also Examples) | | | | | | | | |

### Example 3: Effect of LOXL4-P on solid tumor xenografts

### Animal experiments

Female severe combined immunodeficiency (SCID) bg/bg mice aged 8 to 10 weeks were obtained from Charles River (Sulzfeld, Germany). For the therapeutic response study mice were divided into three experimental groups and one control group. In the first group (n = 12) each mouse was injected *s. c.* in the flank with one million tumor cells from an individual LOXL4-P-sensitive HNSCC cell culture. One month after tumor cell inoculation, if the tumor reached a size of approx. 0,5 cm, each mouse received i.v. injections (200 µg LOXL4-P dissolved in 150 µl PBS, equals 10 mg/kg, which is nearly equivalent to the clinical maintenance dose of cetuximab in humans administered i.v.) every week over a period of up to three and a half months. Animals from the second group (n = 12) were administered with tumor cells in an identical manner to the animals in the first group, except for treatment with LOXL4-P, they received only i.v. injections of 150 µl PBS. In the third group (n = 12) mice were pre-treated i.v. with 200 µg LOXL4-P (dissolved in 150 µl PBS). Immediately after pre-treatment mice were injected *s. c.* in the flank with one million tumor cells from an individual LOXL4-P-sensitive HNSCC cell culture. Tumor size was measured two times per week with a caliper. After the therapeutic response study residual tumors as well as liver, kidney, and spleen were examined histopathologically.
In the control group (n = 5) mice were each injected *s. c.* in their flanks with one million normal epithelial cells. This group was observed over the period of 30 days in order to assure that no tumor growth occurred. The experiments in SCID mice were approved by the Ministry of Environment, Nature and Agriculture of Schleswig-Holstein, Germany.

### Histochemistry

For histochemical analysis xenograft tumors were resected from LOXL4-P-untreated and LOXL4-P treated mice. The tumors were fixed in formalin and embedded in paraffin. Deparaffinized sections (5 µm) were stained with hematoxilin and eosin.

### Statistical analysis

Statistical analysis of the data was performed by means of One-Way ANOVA (SPSS vs 20). Data were considered statistically significant if p ≤ 0,05.

The therapeutic response study involved 3 groups of mice. In the first group (n = 12) mice were s.c. administered each one with tumor cells from an individual LOXL4-P-sensitive HNSCC cell culture. The xenografted tumor cells grew quickly, reaching a tumor size of 0,5 cm within one month. Representative observations regarding the macroscopical and histological appearances of the tumors are presented in Figure 3. The LOXL4-P untreated tumors showed the typical pattern of squamous cell carcinoma. The tumor cells appeared as densely packed aggregates where the cells surrounded a small lumen separated from the cell surface by a distinct internal limiting membrane (Figure 3B and C). For the therapeutic study mice received i.v. injections of 200 µg (10 mg/kg) LOXL4-P (dissolved in 150 µl PBS) every week. Depending on the tumor size, the therapy resulted within 70 to 100 days in extensive tumor destruction as a consequence of the LOXL4-P treatment (Figure 3D - G). Tumor regression occurred as gradual destruction of the tumor tissue. An important indicator for tumor destruction was the development of necrotic patches indicated by dark areas clearly distinguishable from the vital tissue areas. Necrosis progressed with the frequency of LOXL4-P administration. At the end of therapy only a heavily crusted wound remained which did not heal completely. During the experiment and two weeks after therapy mice were observed to assure they did not show any undesired pattern of behavior such as head weaving, suppression of locomotion or reduced climbing activity in comparison to healthy animals not subjected to any experimental procedure. Subsequently, the residual crusted wounds were resected for histological examination (Figure 3H and I). Histological examination of liver and kidney were also carried out without finding any pathological changes.
In the second group (n = 12), mice were administered with tumor cells in an identical manner to the animals from the first group, however, receiving i.v. injections of 150 µl PBS instead of LOXL4-P. As expected, in all these animals tumor development occurred and could not be modified by PBS administration only.
In order to study the effect of LOXL4-P pre-treatment on tumor development, mice (n = 12) were injected i.v. with 200 µg LOXL4-P (dissolved in 150 µl PBS). Immediately thereafter they were injected *s. c.* in the flank with one million tumor cells from an individual LOXL4-P-sensitive HNSCC cell culture. As shown in Figure 4, pre-treatment of animals with LOXL4-P considerably delayed tumor development in contrast to non-LOXL4-P pre-treated animals.

The LOXL4-mAb of the present invention, LOXL4-P, was developed and its potential as a tumor targeting agent in HNSCC cells and xenografts was analyzed. The data show that LOXL4-P is able to target and kill 76% of the tumor cell lines used whereas normal epithelial cells and fibroblasts can tolerate the antibody treatment under same experimental conditions without apparent harm. It is also evident from the cell therapeutic experiments that the mAb did not block the growth and spread of all carcinoma cell lines to the same extent. Depending on cell lines the LD50 values were reached at LOXL4-P concentrations of 15 µg/ml - 45 µg/ml, suggesting that different HNSCC cell lines may have different quantity of LOXL4 antigen on their cell surface. The cause for the different mechanisms of resistance seen in the above mentioned cell lines may also be explained by the fact that these cell lines were derived from parts of the original tumors containing cells resistant to therapeutic agents. These cells may in the presence of a therapeutic agent begin to clonally expand, thereby leading to resistance against the agent as suggested by Diaz et al. (2012).
In preliminary studies, the inventor experimented with a panel of cell lines established from head and neck tumors and realized that some HNSCC cell lines were not able to generate tumors in HNSCC injected animals (unpublished data). Similarly, other cell lines established from malignant HNSCC tissues have frequently been reported to contain variants, which are not able to form tumors when administered into immunosuppressed mice (Rupniak et al., 1985). This might be explained by the fact that the tumor forming efficiency of HNSCC cells is highly anchorage-dependent and associated with the expression of the *ras* oncogene product p21 (Sacks et al., 1988). However, the 17 HNSCC cell lines used in this study have been shown to indeed generate tumors in SCID mice when injected *s. c..*
The LOXL4-mAb of the present invention was shown to exhibit significant enhancement in the treatment efficacy not only *in vitro* but also *in vivo.* As shown in the mouse xenograft therapeutic experiments the antibody, as a single agent, is able to destruct the tumors. Out of the 12 mouse xenografts tested, all (100%) were rated as responsive, whereas no tumor regression occurred in control animals. Depending of the tumor size the therapy had to be continued for a period of up to three and a half months at weekly doses of 200 µg (10 mg/kg) LOXL4-P. During this period, the tumors became progressively necrotic and converted into compact closed fully crusted wounds. The remarkable high response rate of LOXL4-P is a strong call to study the potential mechanism underlying the reactivity of this antibody in more depth. In comparison, the anticancer agent cetuximab targeting the EGFR and is approved for treatment of head and neck cancer (Rivera et al., 2008) was shown to inhibit the growth of 5 out of 8 (63%) xenograft tumors (Krumbach et al., 2011). Assuming that EGFR as a cell surface antigen is strongly overexpressed in head and neck carcinoma, the response rate of cetuximab is rather low. This might be the result of acquired cell resistance to cetuximab associated with the overexpression of *ras*-family members in head and neck cancer cells (Saki et al., 2013). To further investigate the effect of LOXL4-P, SCID mice were first treated with the antibody and were immediately thereafter inoculated with HNSCC cells. Immunoresistance of these mice pre-immunized with LOXL4-P was considerably enhanced indicated by significantly delayed tumor development in comparison to non-LOXL4-pre-treated mice. This, as well as the *in vitro* and xenograft therapeutic experiments, reinforce the demonstration of the anti-tumor effect of LOXL4-P. In conclusion, the potential of LOXL4-P for therapeutic targeting of HNSCC cells and xenografts is described. The findings of the present invention provide strong evidence that the LOXL4 antigene involved in the development and maintenance of the extracellular matrix is a suitable target for mAb therapy of HNSCC.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### REFERENCES

Bou-Assaly W, and Mukherji S. Cetuximab (Erbitux). AJNR Am J Neuroradiol 2010; 31:626 -627
Bradford, MM. A rapid and sensitive for the quantitation of microgram quantitites of protein utilizing the principle of protein-dye binding. Analytical Biochemistry 1976; 72: 248-254
Busnadiego O, González-Santamaría J, Lagares D, Guinea-Viniegra J, Pichol-Thievend C, Muller L, Rodriguez-Pascual F. LOXL4 is induced by transforming growth factor β1 through Smad and JunB/Fra2 and contributes to vascular matrix remodeling Mol Cell Biol 2013; 33:2388-401
Csiszar K, Entersz I, Trackman PC, Samid D, Boyd CD. Functional analysis of the promoter and first intron of the human lysyl oxidase gene. Mol Biol Rep 1996; 23:97-108
Csiszar K. LOX, a novel multifunctional copper amine oxidase family. Prog Nucleic Acid Res Mol Biol 2001; 70:1-32
Csiszar K, Fong SF, Ujfalusi A, Krawetz SA, Salvati EP, Mackenzie JW, et al. Somatic mutations of the lysyl oxidase gene on chromosome 5q23.1 in colorectal tumors. Int J Cancer 2002; 97:636-642
Diaz LA Jr, Williams RT, Wu J, Kinde I, Hecht JR, Berlin J, Allen B, Bozic I, Reiter JG, Nowak MA, Kinzler KW, Oliner KS, Vogelstein B. The molecular evolution of acquired resistance to targeted EGFR blockade in colorectal cancers. Nature 2012; 28:537-540
Erler JT, Bennewith KL, Nicolau M, Dornhofer N, Kong C, Le OT, et al. Lysyl oxidase is essential for hypoxia induced metastasis. Nature 2006; 440:1222-1226
Fernsten PD, Pekny KW, Reisfel, RA and Walker LE. Antigens associated with human squamous cell lung carcinoma defined by murine monoclonal antibodies. Cancer 1986; 46:2970-2977
Gerretsen M, Quakl JJ, Suhl JS, van Walsuml M, Meijer CJLM, Snow GB,van Dongen GAMS, Superior localisation and imaging of radiolabelled monoclonal antibody E48 F(ab'), fragment in xenografts of human squamous cell carcinoma of the head and neck and of the vulva as compared to monoclonal antibody E48 IgG. Br J Cancer 1991; 63:37- 44
Gerretsen M, Schrijvers AHGJ, van Walsum M, Braakhuis BJM, Quak JJ, Meier CJLM, Snow GB, van Dongen GAMS. Radioimmunotherapy of human head and neck squamous cell carcinoma xenografts with 131I-labelled monoclonal antibody E48 IgG. Br J Cancer 1992; 66:496-502
Görögh T, Weise JB, Holtmeier C, Rudolph P, Hedderich J, Gottschlich S, Hoffmann M, Ambrosch P, Csiszar K. Selective upregulation and amplification of the lysyl oxidase-like 4 (LOXL4) gene in head and neck squamous cell carcinoma. J Pathol 2007; 212:74-82
Görögh T, Holtmeier C,Weise JB, Hoffmann M, Ambrosch P, Laudien M, Csiszar K. Functional analysis of the 5' flanking domain of the LOXL4 gene in head and neck squamous cell carcinoma cells. Int J Oncol 2008; 33:1091-1098
Hoffman HT, Karnell LH, Funk GF, Robinson RA, Menck HR. The National Cancer Data Base report on cancer of the head and neck. Arch Otolaryngol Head Neck Surg 1998;124:951-62
Kabat, Sequences of Proteins of Immunological Interest (U.S. Department of Health and Human Services, third edition 1983, fourth edition, 1987, fifth edition 1990)
Kagan HM, Trackman PC. Properties and function of lysyl oxidase. Am J Respir Cell Mol Biol 1991; 5:206-210
Kagan HM and Li W: Lysyl oxidase - properties, specificity, and biological roles inside and outside of the cell. J Cell Biochem 2003; 88: 660-672
Kimmel, K.A.and Carey, T.E. Altered expression in squamous carcinoma cells of an orientation-restricted epithelial antigen detected by monoclonal antibody A9. Cancer Res 1986; 46, 3614-3623
Kirschmann DA, Seftor EA, Fong SFT, Nieva DRC, Sullivan CM, Edwards EM, Sommer P, Csiszar K, Hendrix MJ. A molecular role for lysyl oxidase in breast cancer invasion. Cancer Res 2002; 62:4478-4483
Koprowska I, Zipfel S, Ross AH and Herlyn M. Development of monoclonal antibodies that recognize antigens associated with human cervical carcinoma. Acta Cytol 1986; 30:207-213
Köhler JG, Milstein C. Continuous cultures of fused sells secreting antibody of predefined specificity. Nature 1975; 256:495-497
Krummbach R, Schüler J, Hofmann M, Giesemann T, Fiebeg HH, Beckers T. Primary resistance to cetuximab in a panel of patient-derived tumor xenograft models: Activation of MET as one mechanism for drug resistance. Europ J Cancer 2011; 47:1231-1243
Li W, Nellaiappan K, Strassmaier T, Graham L, Thomas KM, Kagan HM. Localization and activity of lysyl oxidase within nuclei of fibrogenic cells. Proc Natl Acad Sci USA 1997; 94:12817-12822
Mäki JM, Tikkanen H, Kivirikko KI. Cloning and characterization of a fifth human lysyl oxidase isoenzyme: the third member of the lysyl oxidase-related subfamilly with four scavenger receptor cysteine-rich domains. Matrix Biol. 2001; 20:493-496
Mello ML, Contente S, Vidal BC, Planding W, Schenck U. Modulation of ras transformation affecting chromatin supraorganization as assessed by image analysis. Exp Cell Res 1995; 220:374-382
Myoken Y, Moroyama T, Miyauchi S, Takada K, and Namba M. Monoclonal antibodies against oral squamous-cell carcinoma reacting with keratin proteins. Cancer 1987; 60:2927-2937
Parkin DM, Laara E, Muir CS. Estimates of the worldwide frequency of sixteen major cancers in 1980. Int J Cancer 1988; 41:184-197.
Payne SL, Fogelgren B, Fong SFT, Hess AR, Seftor EA, Csiszar K, et al. Lysyl oxidase regulates breast cancer migration and adhesion: implications for FAK/Src signalling pathway. Cancer Res 2005; 15:11429-11436
Peyrol S, Raccurt M, Gerard F, Gleyzal C, Grimaud JA, Sommer P. Lysyl oxidase gene expression in the stromal reaction to in situ and invasive ductal breast carcinoma. Am J Pathol 1997; 150:497-507
Peyrol S, Galateau-Salle F, Raccurt M, Gleyzal C, Sommer P. Selective expression of lysyl oxidase (LOX) in the stromal reactions of broncho-pulmonary carcinomas. Histol Histopathol 2000; 15:1127-1135
Ranken R., White C.F., Gottfried T.G. (1987). Reactivity of monoclonal antibody 17.13 with human squamous cell carcinoma and its application to tumor diagnosis. Cancer Res 1987; 47:5684-5690
Ren C, Yang G, Timme TL, Wheeler TM, Thompson TC. Reduced lysyl oxidase messenger RNA levels in experimental and human prostate cancer. Cancer Res 1998; 58:1285-1290
Rivera F, Vega-Villegas ME, López-Brea MF. Cetuximab, its clinical use and future perspectives. Anticancer Drugs 2008; 19:99-113
Rupniak HT,Rowlatt C, Lane EB, Steele JG, Trejdosiewicz LK, Lakiewicz B, Povey S, Hill BT. Characteristics of four new human cell lines derived from squamous cell carcinoma of the head and neck. J Natl Cancer Inst 1985, 75:621-635
Sacks PG, Parnes SM, Gallick GE, Mansouri Z, Lichtner R, Satya-Prakash KL, Pathak S, and Parsons DF. Establishment and Characterization of Two New Squamous Cell Carcinoma Cell Lines Derived from Tumors of the Head and Neck. Cancer Res 1988; 48:2858-2866
Saki M, Toulany M, Rodemann HP. Acquired resistance to cetuximab is associated with the overexpression of Ras family members and the loss of radiosensitization in head and neck cancer cells. Radiotherapy and Oncology 2013; 108:473-478
Samuel J, Noujaim AA, Willans DJ, Brzezinska GS, Haines DH and Longenecker BM. A novel marker for basal (stem) cells of mammalian stratified squamous epithelia and squamous cell carcinomas. Cancer Res 1989; 49:2465-2470
Tatake RJ, Amin KM, Maniar HS, Jambhekar NA, Srikhande SS and Gangal SG. Monoclonal antibody against human squamous-cell-carcinoma-associated antigen. Int J Cancer 1989; 44:840-845
Weise JB, Rudolph P, Heiser A, Kruse ML, Hedderich J, Cordes C, Hoffmann M, Brant O, Ambrosch P, Csiszar K, Görögh T. LOXL4 is a selectively expressed candidate diagnostic antigen in head and neck cancer. Eur J Cancer 2008a; 44:1323-1331
Weise JB, Csiszar K, Gottschlich S, Hoffmann M, Schmidt A, Weingartz U, Adamzik I, Heiser A, Kabelitz D, Ambrosch P, Görögh T. Vaccination strategy to target lysyl oxidase-like 4 in dendritic cell based immunotherapy for head and neck cancer. Int J Oncol 2008b; 32:317-322

## Claims

1. An antibody or antigen-binding fragment thereof that binds to an epitope of anti-lysyl oxidase-like 4 (LOXL4) in the cytoplasm and/or on the cell surface of tumor cells, for use in the treatment of a tumor which expresses LOXL4.

2. The antibody for use according to claim 1, wherein said epitope comprises or consists of the amino acid sequence KVWDLKMR (SEQ ID NO: 1).

3. The antibody for use according to claim 1 or 2, which is a monoclonal antibody, preferably monoclonal antibody LOXL4-P as produced by hybridoma LOXL4-P, deposited with the DSMZ-Deutsche Sammlung von Mikroorganismen und Zellkulturen GmbH, Mascheroder Weg 1b, D-38124 Braunschweig, Germany on 19. March 2014, and assigned Accession Number DSM ACC3233 or an antibody capable of competing with monoclonal antibody LOXL4-P for binding LOXL4 in the cytoplasm and/or on the cell surface of tumor cells.

4. The antibody or antigen-binding fragment for use according to any one of claims 1 to 3, which is capable of causing cell death and cell lysis as based on the measurement of LDH released from the cytosol of damaged cells.

5. The antibody for use according to any one of claims 1 to 4, which is a human, humanized, xenogeneic, or a chimeric human-murine antibody.

6. The antigen-binding fragment for use according to any one of claims 1 to 5, which is selected from the group consisting of a single chain Fv fragment, an Fab' fragment, an Fab fragment, and an F(ab')₂ fragment.

7. A bifunctional molecule that comprises the antigen-binding domain of an antibody as defined in any one of claims 1 to 6 and at least one further functional domain for the treatment of a tumor which expresses LOXL4.

8. The bifunctional molecule of claim 7, which is a bispecific antibody and/or wherein said further functional domain is a cytotoxic agent or a label.

9. A pharmaceutical composition comprising the antibody or antigen-binding fragment as defined in any one of claims 1 to 6 or the bifunctional molecule of claim 7 or 8 and further comprising a pharmaceutically acceptable carrier, for use in the treatment of a tumor which expresses LOXL4.

10. The pharmaceutical composition for use according to claim 9, further comprising an immune stimulatory agent.

11. An anti-LOXL4 antibody as produced by hybridoma LOXL4-P, deposited with the DSMZ-Deutsche Sammlung von Mikroorganismen und Zellkulturen GmbH, Mascheroder Weg 1b, D-38124 Braunschweig, Germany on 19. March 2014, and assigned Accession Number DSM ACC3233, or an antibody or antibody fragment comprising at least one complementarity determining region (CDR) of the anti-LOXL4-P antibody as produced by hybridoma DSM ACC3233 and being capable of binding to an epitope of LOXL4 in the cytoplasm and/or cell surface of tumor cells, wherein said epitope comprises or consists of the amino acid sequence KVWDLKMR (SEQ ID NO: 1).

12. A polynucleotide encoding at least the variable region of one immunoglobulin chain of the antibody or antigen-binding fragment of claim 11.

13. A vector comprising the polynucleotide of claim 12.

14. A host cell comprising a polynucleotide of claim 12 or a vector of claim 13, preferably wherein the host cell is hybridoma DSM ACC3233.

15. The antibody or antigen-binding fragment for use according to any one of claims 1 to 6, the bifunctional molecule of claim 7 or 8, the pharmaceutical composition for use according to claim 9 or 10, or the antibody of claim 11, wherein the tumor is selected from the group consisting of head, neck, larynx, esophagus, stomach, lung, liver, colon, rectum, pancreas, breast, ovary, uterus, cervix, vulva, penis, bladder or kidney tumors, and metastatic cells in general.


SEQUENCE LISTING

<110>  Christian-Albrecht University

<120>  Therapeutic and diagnostic anti-LOXL4 antibodies

<130>  CH41A12/P-EP

<160>  1

<170>  PatentIn version 3.5

<210>  1
<211>  8
<212>  PRT
<213>  Artificial Sequence

<220>
<223>  LOXL4 epitope

<400>  1

Lys Val Trp Asp Leu Lys Met Arg
1               5